# EUROPEAN PATENT SPECIFICATION

(11) **EP 1 539 073 B1**
(45) Date of publication and mention of the grant of the patent: **06.04.2011**
(21) Application number: 03762948.2
(22) Date of filing: 30.06.2003
(51) Int. Cl.: A61F 13/475, A61F 13/491

(54) **ABSORBENT PRODUCT FOR MEN**
SAUGFÄHIGES PRODUKT FÜR MÄNNER
PRODUIT ABSORBANT POUR HOMME

(30) Priority: 05.07.2002 SE 0202102
(43) Date of publication of application: 15.06.2005
(73) Proprietor: SCA Hygiene Products AB, 405 03 Göteborg (SE)
(72) Inventor: SANDIN, Cécile, S-431 36 Mölndal (SE); STRANNEMALM, Kenneth, S-448 31 Floda (SE); ELFSTRÖM, Anna-Carin, S-423 38 Torslanda (SE)
(74) Representative: Olsson, Stefan
(86) International application number: PCT/SE2003/001137
(87) International publication number: WO 2004/004617

(56) References cited:
- WO-A1-96/05786
- WO-A1-99/42066
- US-A- 5 486 168
- US-A- 5 558 734
- US-A- 5 810 799

## Description

### TECHNICAL FIELD

The present invention relates to an absorbent product for men, comprising an absorption body which tapers preferably towards one end from a front section of the product towards a crotch section of the product and which is enclosed in a sheath consisting of a liquid-tight layer on the side of the absorption body facing away from the user during use and a liquid-permeable layer on the opposite side of the absorption body, both of which layers extend beyond the absorption body round about this and are mutually joined there, and the absorption body is arranged to extend, during product usage, from the said front section of the product in the direction of the crotch region and is intended to extend with its narrower end section to slightly below the penis of the user.

### BACKGROUND ART

In practice, conventional nappies have hitherto commonly been used as incontinence pads for men. The drawback with conventional nappies is that they are intended to absorb both urine and faeces and that they are therefore not suitable for men who only need a urine-collecting pad. Conventional nappies are relatively cumbersome, having thick and wide absorption bodies which, during usage, extend the whole of the way from the abdominal section of the user, through the crotch and a substantial way up over the back of the user. Large and bulging nappies are not, of course, suitable, especially not for men who have urine incontinence but who are not in any way handicapped. For reasons of both comfort and modesty, there is a great need for more purposeful products for incontinent men.

Light incontinence is a large and hidden handicap from which many men suffer and which seriously limits their chance of leading a normal active life. A large group of men who suffer from this are those with prostate problems. Following surgery, these men are usually afflicted by drip incontinence, which causes a great deal of suffering.

Incontinence pads for men with light incontinence are previously known per se, but they have not worked satisfactorily in all respects. Examples of previously known incontinence pads for men are pads of the type which have a container-like part for receiving the genitals of the male user. Fundamental drawbacks with these are that they are altogether too warm, at the same time as they are too tight-fitting and hence uncomfortable for the user. A further drawback with pads of this type is that they are too rigid and can give rise to chafing.

Swedish patent specification SE 450 811 describes an incontinence pad intended for men, consisting of an upper shield-like part, which, during usage of the pad, is applied over the penis and scrotum of the user, and a lower part, which, during usage of the pad, curves inwards under the penis and scrotum without totally surrounding these and has a downward-tapering cup-like shape. The pad according to the said publication is airy and relatively comfortable compared with conventional nappies. Unlike the abovementioned incontinence pads having a container-like part, nor does it enclose the organs of the user, but instead forms an airy splash guard. The configuration of the pad with a downward-tapering, cup-like part provides, in addition to good adjustment of the pad to the body of the user, at the same time a guard for downward splashes.

The pad according to the said publication is formed from a flat blank, the one end of which has two separate longitudinal flaps. The cup-like lower part of the pad is obtained by the said flaps being curved and mutually connected in somewhat overlapping arrangement.

A drawback with these previously known incontinence pads is that the production process is relatively complicated with cutting steps, folding steps and connecting steps. Another drawback is that the pad, in order to work fairly reliably, should have a good fit for the respective user, which, with this design, is unattainable in practice since the size of different users naturally varies considerably and it is not economically possible to have a satisfactory range of sizes. A further drawback is that the incontinence pad according to SE 450811, in order to be able to assume the desired cup-like shape at the bottom, limits the choice of material in the pad, since this must have relatively high stiffness in order to assume and maintain the desired cup shape.

Another example of a previously known incontinence pad for men is described in US 5 486168. This pad has an absorption body which tapers towards one end from a front section of the product towards a crotch section of the product and which is enclosed in a sheath consisting of a liquid-tight layer on the one side of the absorption body and a liquid-permeable layer on the opposite side of the absorption body. The two layers extend beyond the absorption body and are mutually joined there. The incontinence pad according to the last-named publication is provided with elastic threads or the like, which are applied with pretensioning to the sheath on both sides of the absorption body and which converge in the direction of the narrower end section of the absorption body. The absorption body has lower flexural stiffness transversely to the transverse direction the closer one gets to the narrow end of the absorption body and the elastic threads will therefore lend the absorption body increased curvature the closer one gets to the narrow end. The elastic and the tapered absorption body interact to give the pad a cup-like part at the bottom, which cup-like part, during usage of the pad, is intended to arc in under the penis and scrotum of the user.

In a configuration according to US 5 486168, it is important for the absorption body to have a stiffness which enables the absorption body to interact with the elastic as intended, thereby limiting the choice of materials for the absorption body. A further drawback with the design according to the last-named patent publication is that any variances in stiffness per unit of area over the surface of the absorption body can give rise to unwanted folds in the lateral direction out from the absorption body, which in tum can lead to urine leakage along the folds in the lateral direction out from the product. Another drawback is that incontinence pads according to US 5 486168 are difficult to pack in the compressed state, since there is a high risk of the three-dimensional products formed by the elastic being destroyed in the packaging operation or during storage in the packaging. The tension from the elastic of the absorption body, in combination with external mechanical influences, results in both the elastic and the absorption body being subjected to high stresses.

US 5 810 799 discloses a diaper with a barrier layer applied on the liquid permeable layer. The barrier layer is positioned above the tip of the penis when the diaper is worn. US 5 810 799 concerns baby diapers and the liquid barrier is intended to block urine sprayed in an upward direction. The natural inclination of the baby's penis is to point upwards towards the baby's head when the baby is diapered.

There has long been a great need for discrete, comfortable and effective incontinence pads for men with light incontinence, such as drip incontinence. No products have emerged which are satisfactory in every respect. There is a large known group of men with light incontinence who would need more practical products to be able to lead a normal active life without being fearful of urine leakage and feeling that the pads which are wom are visible or perceptible outside the clothes of the user.

It is also known that there is a very large group of men who have not sought care for their incontinence problems, and whose problems are not related to previous surgery. The size of this group is not known, but studies in certain countries show that light incontinence is a very widespread problem and that the need for efFective, discrete and comfortable products is very great

### DISCLOSURE OF INVENTION

As a result of the present invention, a product of the type stated in the introduction has been produced, by means of which the above-stated problems have been totally eliminated. To this end, the product according to the invention is primarily characterized in that on the liquid-permeable layer, at the narrower end section of the absorption body, a liquid barrier is applied, said liquid barrier is positioned such that during usage, it is situated below the penis of the user and is curved and convex in the direction of the crotch section to prevent urine emitted by the user from leaking from the surface of the absorption body towards the crotch region of the user.

According to one embodiment, the invention is characterized in that the said absorbent product is constituted by an insert intended for use in combination with underpants and in that, to this end, the product is provided with one or more fastening members on the outer side of the liquid-tight layer, which fastening members are intended to hold the absorbent product in its intended place inside the pants.

According to another embodiment, the invention is characterized in that the product as a whole has a pants-like shape having a front section, a rear section and a crotch section, the front section and rear section being intended to surround the waist of the user, and the front and/or rear section is provided with waist elastic, which is intended to hold a applied product in place on the user.

According to another embodiment, the invention is in this case characterized in that the front section has at least one elastic member, which, during product usage, enables the front section of the product to be pulled down, counter to the action of the said elastic member, to a position in which the upper limit edge of the front section in the middle region of the front section is situated below the penis of the user, at the same time as the upper limit edge of the front section in the two outer edge regions of the front section is arranged to be held in place around the waist of the user, in that the absorbent material is configured with one or more deformation zones, which enable those parts of the absorption body which, during product usage, are situated above and over the penis of the user to be drawn down together with the rest of the front section when the front section of the product is pulled down, and in that the front section of the product and the absorption body are arranged to be returned by the elastic member to their original usage position.

In previously known incontinence pads for men, the user has, in practice, been unable to visit urinals. It is naturally inconceivable for a man, in connection with a toilet visit at a urinal, to pull down a whole incontinence pad and thereby reveal private and embarrassing problems to those around him. With a pad according to the last-named embodiment, this problem has been eliminated.

According to a further illustrative embodiment, the invention is characterized in that the said waist elastic is formed from an elastic first piece which, in the extended state, is essentially rectangular and which is intended to partially surround the trunk of the user and form the rear section and side sections of the pants-like product, in that a second piece, incorporated in the product, is configured to form the front section and crotch section of the pants-like product, in that the said second piece is elongated with two opposing end edges and two opposing longitudinal edges, in that the width of the second piece, at least at the crotch section, is less than the length of the first piece, in that the second piece with its longitudinal direction is arranged perpendicularly to the longitudinal direction of the first piece and is connected by a first end section to the one longitudinal edge section of the first piece and centrally on this, in that the one end section of the first piece is connected to a first side edge section of the second piece, and in that the second end section of the first piece is correspondingly connected to a second side edge section of the second piece, and the absorbent element is applied, in its entirety, on the second piece.

By virtue of the fact that the whole of the rear section is elastic and, together with the elastic side sections, forms a single elastic continuous first piece, the pants as a whole are very responsive to body movements. Local disturbances are absorbed and smoothed out by the continuous elastic piece and are not transmitted to the less resilient parts of the front section and crotch section situated directly in front of the genitals of the user. Compared with conventional nappies and previously known pants-shaped absorbent products, with the product according to the present invention a superior fit and comfort are obtained. The greater part of the pants-like garment remains totally plain. The seams which are needed are applied at the transition between the front section and the first piece and at the transition between the first and second piece at the crotch section. These seams end up in places which are not subjected to any great pressure during product usage and there is less risk of chafing and pressure sores caused by seams on the pants-like product.

The configuration of the absorbent product according to the invention from only two part-pieces and in which the absorption body is situated entirely on the second part-piece offers increased freedom in the choice of production method compared with previously known pants-shaped products. The prospect of freely choosing suitable materials on different parts of the absorbent product is also substantially increased compared with conventional pants-like absorbent products produced with outer layers configured in a single piece.

According to one embodiment, the invention is characterized in that the said liquid barrier is arranged to follow the contour of the lower, narrower end section of the absorption body and is applied in its entirety inside the said contour.

According to another embodiment, the invention is characterized in that the liquid barrier crosses the absorption body close to its narrower end.

According to a further embodiment, the invention is characterized in that the liquid barrier extends in the lateral direction beyond the absorption body and in the transverse direction spans the whole of the absorbent product. This configuration can be simpler from the production aspect compared with a design in which the liquid barrier only crosses the actual absorption body.

According to another embodiment, the invention is characterized in that the liquid barrier, at least during product usage and at least close to the middle of the absorption body in the transverse direction, has a height of at least 5 mm, preferably 10-20 mm. The liquid barrier can therefore, within the scope of the invention, always assume the said height or be configured such that the liquid barrier is raised when the user dons the product and, in so doing, bends it somewhat.

According to one embodiment, the invention is characterized in that the liquid barrier is fixed in the rest of the absorbent product only along its outer edge section and in that inner-situated sections of the liquid barrier are arranged to be raised from the liquid-permeable layer during product usage.

According to another embodiment, the invention is characterized in that the liquid barrier is constituted by a roll formed from one or more band-shaped materials, which roll is bent into the desired convex shape in the direction of the narrower end of the absorption body.

According to another embodiment, the invention is characterized in that the liquid barrier is constituted by a number of circumferential folds of one or more band-shaped materials, which liquid barrier, following the formation of the said folds, is elongated in the direction of the fold lines forming the folds, and in that the liquid barrier is folded or bent into the desired shape.

According to one embodiment, the invention is in this case characterized in that the elongated liquid barrier is folded in the middle along a transverse oblique line, such as at an angle of 45°, in relation to the longitudinal direction to form a V-shaped liquid barrier with the point of the V against the crotch section.

According to a somewhat modified embodiment, the invention is characterized in that the elongated liquid barrier is folded at two places along transverse lines along an acute angle, viewed from the lower limit edge of the liquid barrier in the applied position, to form an essentially U-shaped liquid barrier with the base of the U against the crotch section.

According to one embodiment, the invention is characterized in that the band material incorporated in the liquid barrier is constituted by non-woven, preferably hydrophobic non-woven material, or by a laminate of non-woven material and a plastics film.

According to another embodiment, the invention is characterized in that the liquid barrier is constituted by a single material strip, for example a hydrophobic non-woven material, which has been folded or bent into suitable shape before being applied.

According to a further embodiment, the invention is characterized in that the liquid barrier, on its free longitudinal edge section, is provided with a pretensioned longitudinal elastic element, such as an elastic thread, which element is intended to hold the liquid barrier in the raised state during product usage.

According to another embodiment, the invention is characterized in that the liquid barrier has an inherent stiffness of such magnitude that a liquid barrier applied in the folded or bent state, by virtue of its own inherent stiffness in the said bent or folded state during product usage, is held with its free longitudinal edge section in the raised state.

According to a further embodiment, the invention is characterized in that the absorption body is formed from cellulose fluff, possibly with highly absorbent material mixed in, and in that the liquid barrier is constituted by a moulding, which is formed from air-laid fibres such as cellulose fluff, and in that this moulding is applied so that it follows the contour of the narrower end section of the absorption body on or directly outside this.

According to another embodiment, the invention is characterized in that the liquid barrier is constituted by a foam material which has been cast or folded into the desired shape.

### BRIEF DESCRIPTION OF DRAWINGS

The invention will be described in greater detail below with reference to illustrative embodiments shown in the appended drawings, in which:
- Figure 1: shows diagrammatically in flat form a phase in the development of an absorbent product according to a first embodiment of the invention.
- Figure 2: shows diagrammatically in flat form an assembled product according to the first embodiment.
- Figure 3: shows diagrammatically in flat form a phase in the development of an absorbent product according to a second embodiment of the invention.
- Figure 4: shows diagrammatically in flat form an assembled product according to the second embodiment.
- Figure 5: shows in flat form a third embodiment of the product according to the invention.
- Figure 6: shows in flat form a fourth embodiment of the product according to the invention.
- Figure 7: shows in flat form a fifth embodiment of the product according to the invention.
- Figure 8: shows diagrammatically an illustrative embodiment of a liquid barrier incorporated in the product according to the invention.
- Figures 9-9d: show various phases in the development of a liquid barrier according to another illustrative embodiment.
- Figure 10: shows diagrammatically a third illustrative embodiment of a liquid barrier in a product according to the invention.
- Figure 11: shows diagrammatically a fourth illustrative embodiment of a liquid barrier in a product according to the invention.
- Figure 12: shows diagrammatically in perspective a further embodiment of the liquid barrier and in the state of use.
- Figure 13: shows diagrammatically a phase in the development of a further embodiment of the product according to the invention.
- Figure 14: shows diagrammatically in flat form an assembled product according to the embodiment shown in Figure 13.
- Figure 15: shows diagrammatically and in flat form another embodiment of a product according to the invention.

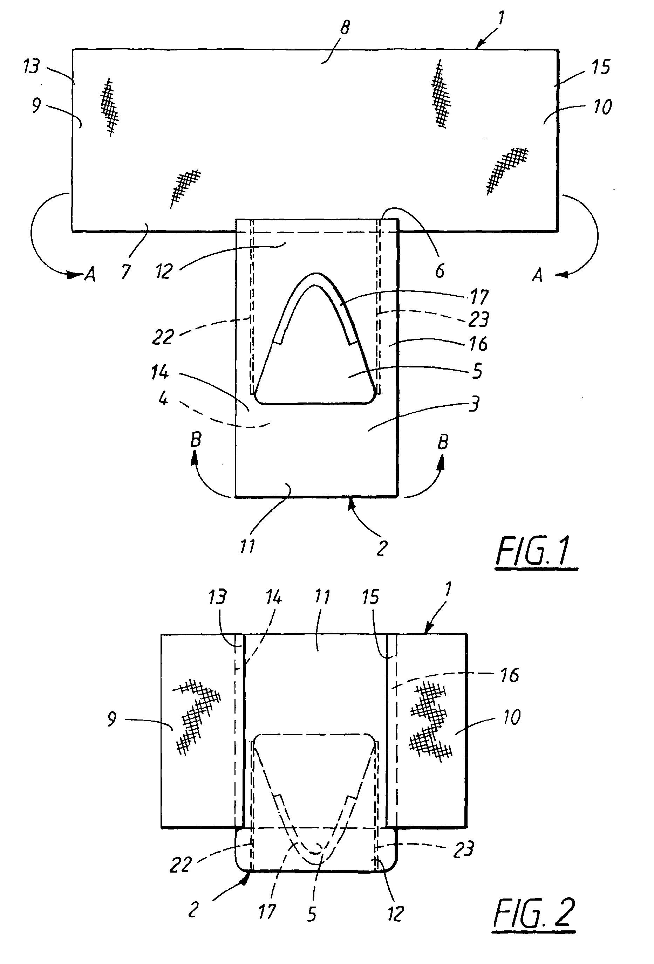

As can be seen from Figure 1, the product in the embodiment according to Figure 1 comprises a first piece 1. This is elastically stretchable and is shown in Figure 1 in flat and evenly extended state, in which the elastic piece 1 is essentially rectangular. The elastic first piece 1 can be formed from conventional materials familiar to the person skilled in the art, such as woven elastic materials, elastic non-wovens or elastic films. The essential point is that the elastic piece is stretchable for a user when putting on the product and that it fits tight with suitable tightening around the user when the article is worn. The tightening is adjusted, of course, by means of size and elastic stretchability.

The product incorporates a second piece 2, consisting of an inner layer 3, an outer layer 4 and an absorption body 5 applied between these. In the embodiment shown in Figure 1, this is in flat form essentially triangular. The choice of material in the absorption body is not critical but can be chosen from amongst materials or material combinations familiar to the person skilled in the art. For example, the absorption body can be made up of cellulose fluff with superabsorbent material in powder or fibre form mixed in with this. The outer layer 4 can consist, for example, of a polyethylene film of a type conventionally used for absorbent products. A liquid-tight film in combination with an outer fibre layer is suitable if an absorbent product having a more textile-like appearance is sought. The inner layer 3 can be formed from a liquid-permeable non-woven material. The outer layer and the inner layer extend with sections beyond the absorption body and are mutually connected in these sections. The second piece 2 is elongated and is applied with its longitudinal direction perpendicular to the longitudinal direction of the first piece 1. The second piece 2 is connected by an end section 6 to the one longitudinal edge section 7 of the first piece 1 and centrally on this. The connection can be realized, for example, by the use of bonding agent, heat bonding or ultrasonic bonding.

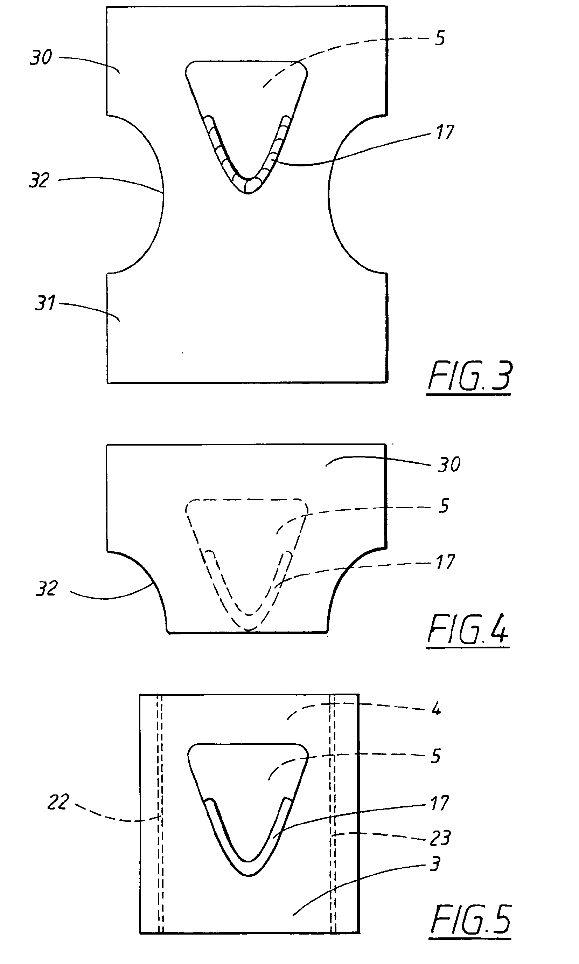

In Figure 1, the arrows A and B are used to illustrate how the first and second pieces are folded for the formation of the pants-shaped absorbent product shown in Figures 2 and 3. The elastic piece 1 is folded in according to the shown arrows A to form the rear section 8 and side sections 9, 10 of the products, whereas the second piece is folded upwards according to the arrows B to form the front section 11 and crotch section 12 of the product.

The one end section 13 of the first piece is applied in slightly overlapping arrangement with a first side edge section 14 of the second piece and the second end section 15 of the first piece is correspondingly applied in slightly overlapping arrangement with a second side edge section 16 of the second piece. These overlapping parts are mutually connected, for example by means of bonding agent, heat bonding or ultrasonic bonding. Alternatively, the front section and the crotch section can be detachably applied along the said overlapping sections 13, 14 and 15, 16 respectively. A detachable connection of this kind can be constituted, for example, by a hook member (not shown), whereby the pants-like product can be opened and subsequently closed up again with the same fit and tightening.

On the liquid-permeable layer 3, at the narrower end section of the absorption body, a liquid barrier 17 is applied. In the illustrative embodiment shown in Figures 1 and 2, this is arranged to follow the contour of the lower, narrower end section of the absorption body and is applied in its entirety inside the said contour. The liquid barrier 17 is arranged to prevent urine emitted by the user from leaking from the surface of the absorption body towards the crotch section 12.

As stated above, the absorption body can be formed, for example, from cellulose fluff, possibly with highly absorbent material mixed in. The liquid barrier can be constituted by a moulding, which is formed from air-laid fibres such as cellulose fluff. As shown in Figures 1 and 2, this moulding is expediently applied so that it follows the contour of the narrower end section of the absorption body and on top of its surface. The liquid barrier can also be applied outside the absorption body and is in this case expediently applied directly adjacent to the contour of the absorption body.

The liquid barrier can be configured in many different ways, as will be evident from the continued description. It has proved expedient for the liquid barrier, at least during product usage and at least close to the middle of the absorption body in the transverse direction, to have a height of at least 5 mm, preferably 10-20 mm. For the product to work as intended, the liquid barrier needs to be positioned such that, during product usage, it is situated below the penis of the user. The liquid barrier must also be curved and convex in the direction of the crotch section 12. The fact that the barrier has a necessary height of at least 5 mm and is convexly curved in the direction of the crotch section means that the pad is very secure against leakage and liquid is prevented from passing the liquid barrier 17 in the direction of the crotch section or in the lateral direction in which the liquid barrier with its upwardly curved sections constitutes an effective barrier.

In the embodiment according to Figures 1 and 2, the pants-like product is provided with pretensioned leg elastic 22, 23 in the crotch section, the leg elastic being intended, during product usage, to bring the side flaps, formed by the outer layer and the inner layer, on both sides of the absorption body into bearing contact against the leg of the user in his crotch region.

In Figures 3 and 4, another embodiment of a pants-like product is shown. The absorption body 5 and the liquid barrier 17 are configured in the same way as has been described above in connection with Figures 1 and 2. As can be seen from Figure 3, the sheath for the absorption body, formed by the outer layer 4 and the inner layer 3, is shaped like an hourglass, having a front section 30, a rear section 31 and a crotch section 32. The side sections of the sheath in the front section are connected in a conventional manner to opposing side sections on the rear section to form pants according to Figure 4. The sheath of the pants is conventionally configured with waist elastic (not shown) in order to hold the applied pants in place on the user and with leg elastic (not shown) for snug tightening of the sheath against the leg of the user in the crotch region.

The liquid barrier 17 can be formed together with the absorption body from air-laid fluff and applied inside the sheath. The liquid barrier and the absorption body can also be configured in one piece from a cast foam material, possibly with highly absorbent material mixed in. It can, of course, be an alternative to choose in this context a foam material which has very high absorbency and high holding capacity even under pressure loading. If the liquid barrier does not contain any direct liquid-barring layer, then high holding capacity is important.

A pants-like product according to Figures 3 and 4, instead of having a liquid barrier configured in one piece with the absorption body, can have a liquid barrier on the outer side of the inner layer 3. Liquid barriers of this type will be described in greater detail below in connection with a number of illustrative embodiments.

The absorbent product according to the invention does not need to be in the form of complete disposable pants, but can be constituted by a separate insert which can be used in combination with normal textile pants or in combination with special pants, matched to the insert, for repeated use.

An example of such an insert is shown in Figure 5. The insert has an absorption body 5 enclosed in a rectangular sheath, consisting of a liquid-tight outer layer 4 and a liquid-permeable inner layer 3. To the outer side of the liquid-tight layer 4 are applied one or more fastening members (not shown) for detachable connection to a pair of interacting underpants. In the illustrative embodiment shown, the sheath is provided with pretensioned elastic 22, 23 on both sides of the absorption body. The liquid barrier 17 is V-shaped and applied so that it follows the contour of the absorption body 5.

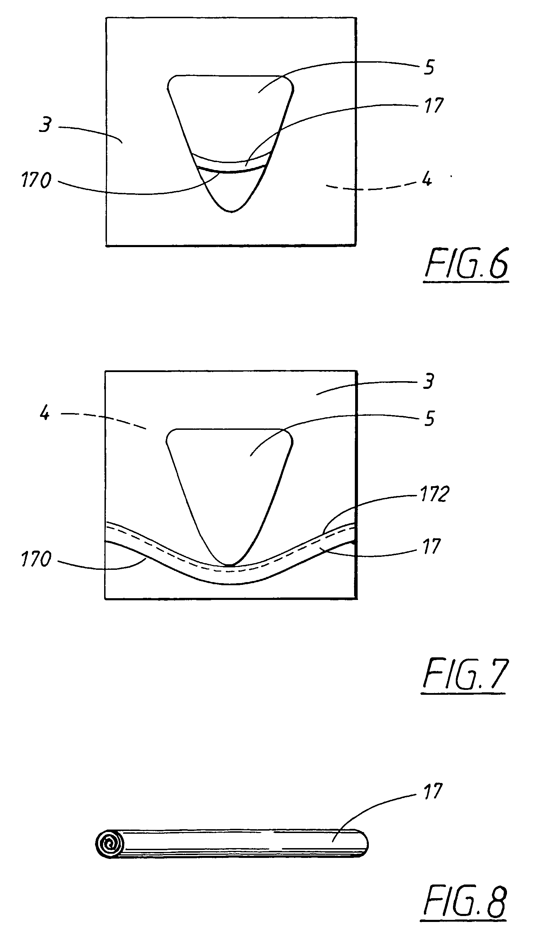

In Figure 6, an embodiment is shown which is somewhat modified in relation to the embodiment according to Figure 5. The fundamental difference is that the liquid barrier 17 here has less curvature and is applied higher up on the absorption body 5. A lesser curvature means that a liquid barrier which is somewhat stiff can be more easily connected to the surface material, i.e. the inner layer 3. In the illustrative embodiment shown, the liquid barrier 17 can be constituted by a material strip which is flat in its basic form and is somewhat stiff and which is only fixed to the surface material along its lower, outer edge section 170. The liquid barrier will hence during product usage, since the fastening of the edge section 170 follows a curved path, lift up from the surface material and form a vertical liquid barrier. The liquid barrier 17 has here been shown applied higher up than in the illustrative embodiments above. One reason for this is that the liquid barrier must have a certain extent in the lateral direction in order to form an effective protection against liquid dispersion.

In Figure 7, an illustrative embodiment is shown which is somewhat modified in relation to the embodiment according to Figure 6. Here, too, the liquid barrier 17 has less curvature than the liquid barriers in the embodiments according to Figures 1-5. In the embodiment according to Figure 7, the liquid barrier 17 extends across the full extent of the insert in the lateral direction, thereby simplifying the product process in terms of the fitting of the liquid barrier. This is only fixed in the inner layer 3 along its lower, outer edge section 170 and is provided along its free edge section with a pretensioned elastic thread 172, which is intended to hold the liquid barrier in the raised state. In the embodiment shown in Figure 7, it is, of course, important for the outer layer 4 to be liquid-tight.

In Figure 8, an illustrative embodiment is shown of a liquid barrier for a product according to the invention. The liquid barrier 17 is constituted by a roll formed from one or more band-shaped materials. For example, the roll can be formed from a hydrophobic non-woven material. Alternatively, the roll can be formed from a plastics film. A further example is a laminate of a liquid-tight film and a non-woven material. The roll is expediently so configured and consists of material of such a type that the roll is flexible and can be bent into the desired convex shape in the direction of the narrower end of the absorption body and applied to or in connection with the absorption body.

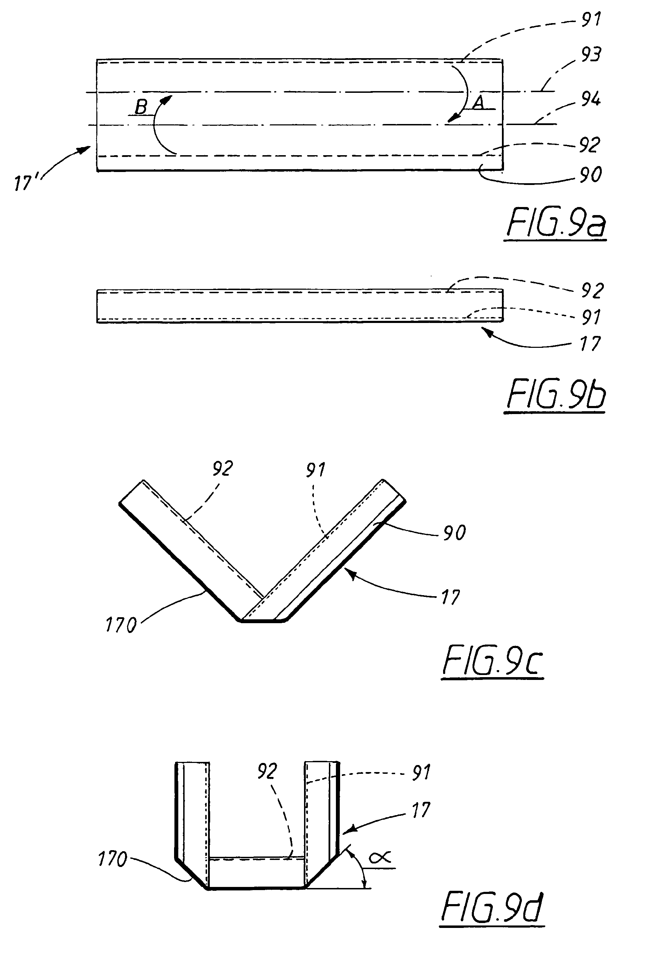

Figures 9a-d show an illustrative embodiment of a liquid barrier for a product according to the invention and how the liquid barrier is made. Figure 9a shows the basic material in the form of an elongated material piece consisting of, for example, hydrophobic non-woven material or a laminate of two or more layers. The material piece 17' is intended to be triple-folded into circumferential folds and in the illustrative embodiment shown has an edge section 90, which edge section, after the material piece is folded, is intended to be connected to the outer side of the folded material piece. The material piece in Figure 1 has been provided with two longitudinal elastic threads 91, 92, which are applied in the pretensioned state, as shown in Figure 9a, with one elastic thread 91 along the one edge section and with the other elastic thread 92 directly in front of the edge section 90. The material piece 17' is folded first along a longitudinal fold line 93, as shown by the arrow A, and then along a longitudinal fold line 94, as shown by the arrow B. The edge section 90 is next connected to the outer side of the folded material piece. The folded material piece 17' is shown in Figure 9b. Following the folding, the elastic threads 91, 92 are situated along opposing edge sections. For the formation of the V-shaped liquid barrier 17 shown in Figure 9c, the material piece 17' in Figure 9b is folded in the middle at a 45° angle. This angle can, of course, be varied in order to obtain the desired inclination of the arms of the V-shaped liquid barrier.

The V-shaped liquid barrier according to Figure 9c is intended to be connected along the whole of its length by its lower outer edge section 170 to the inner layer of the absorbent product and expediently along the contour of the lower part of the absorption body, as shown in the embodiment according to Figure 5. The elastic sections which are situated at the edge sections connected to the inner layer of the absorbent product will become inactive as a result of the connection and only the elastic sections along the free edge sections of the liquid barrier continue to be active in raising the liquid barrier. Those edge sections 170 of the liquid barrier which are connected to the inner layer 3 have been marked in Figure 9c with bolder lines.

Alternatively, a material strip 17' according to Figure 9b can be folded into a U-shape, as shown in Figure 9d. The fold angle α is in the drawing 45°. This angle can be reduced if a liquid barrier is desired in which the arms of the U-shape are more outwardly inclined. The U-shaped liquid barrier is intended to be connected along the edge sections 170 marked in the drawing with thicker lines. Only those pieces of the elastic threads 91, 92 which are marked in Figure 9d will be active in raising the liquid barrier. Other sections have been disabled as a result of the connection to the inner layer 3.

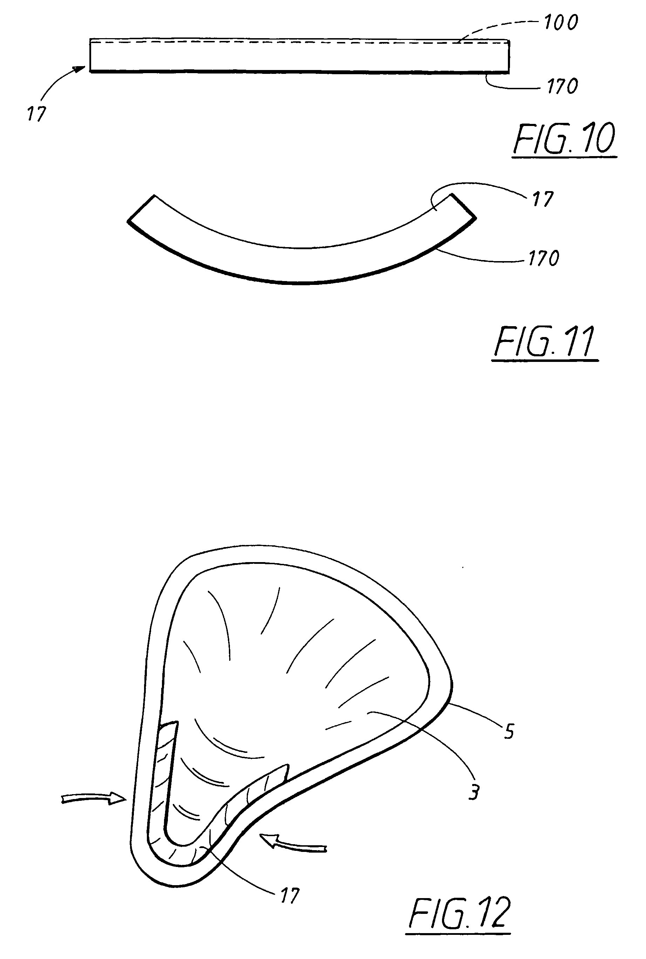

In Figure 10 a liquid barrier in the form of a single elongated material strip is shown, for example made of hydrophopic non-woven material, which is intended to be folded or bent into suitable shape prior to being applied to the inner layer 3 of a product according to the invention. The elongated material strip can also be constituted by a liquid-tight foam material. The material strip can be provided with a pretensioned longitudinal elastic thread 100, which is intended to hold the liquid barrier in the raised state. If the material in the elongated strip has a certain stiffness and the material strip is fastened along its lower edge section 170, marked in the drawing with a thicker line, the elastic thread 100 is unnecessary in the curved state, since the barrier is raised by its own stiffness, firstly by a bit after the material strip is fastened and secondly by a further bit when the product as a whole is bent as it is applied to the user.

Figure 11 shows a curved material strip cut out in flat form and made of, for example, a plastics film, a foam material or a laminate of non-woven material and a plastics film. The material strip is intended to be connected to the outer layer 3, for example as shown in Figure 6, along the edge sections 170 marked in Figure 11 with thicker lines. Expediently, the material strip has such inherent stiffness that the liquid barrier formed by the strip is raised when the product is taken into use and applied to the user.

Figure 12 illustrates how a liquid barrier 17 in the form of a flat material strip is intended to perform in the usage position when the leg of the user, in his crotch section, presses on the absorption body in the direction of the arrows in Figure 12. The liquid barrier 17 is raised and prevents urine emitted by the user from leaking out into the crotch region.

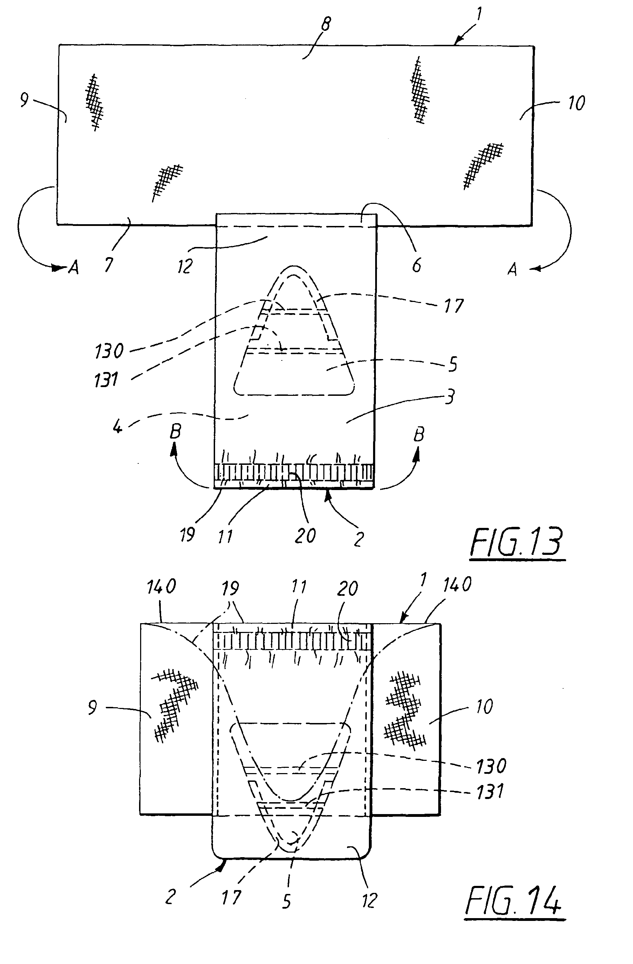

Figures 13 and 14 show a pants-like product which has much in common with the embodiment according to Figures 1 and 2. Components identical to or corresponding to similar parts as in the embodiment according to Figures 1 and 2 have in Figures 13 and 14 been provided with the same reference notations. The embodiment according to Figures 13 and 14 differs from the embodiment shown in Figures 1 and 2 by the fact that the front section has been provided with at least one elastic member 20, which, during product usage, enables the front section 11 of the product to be pulled down, counter to the action of the said elastic member 20, to a position in which the upper limit edge 19 of the front section in the middle region of the front section is situated below the penis of the user, at the same time as the upper limit edge of the front section in the two outer edge regions 140 of the front section is arranged to be held in place around the waist of the user. The absorbent material is configured with one or more deformation zones 130, 131, which enable those parts of the absorption body which, during product usage, are situated above and over the penis of the user to be drawn down together with the rest of the front section when the front section of the product is pulled down. The front section of the product and the absorbent element are arranged to be returned by the elastic member to their original usage position.

In previously known incontinence pads for men, the user has, in practice, been unable to visit urinals. It is naturally inconceivable for a man, in connection with a toilet visit at a urinal, to pull down a whole incontinence pad and thereby reveal private and embarrassing problems to those around him. With a pad according to the embodiment shown in Figures 13 and 14, this problem has been eliminated. In Figure 14, the dash-dot line 19 has been used to illustrate the extent to which the front section of the product is pulled down in connection with a visit to a urinal. In the illustrative embodiment shown, the deformation zones 130 and 131 are constituted by two fold notches. When the front section 11 of the product is pulled down, the absorption body 5 is folded firstly along the deformation zone 130 and then, upon continued pulling down, along the deformation zone 131. The liquid barrier characteristic of the present invention has been denoted by 17.

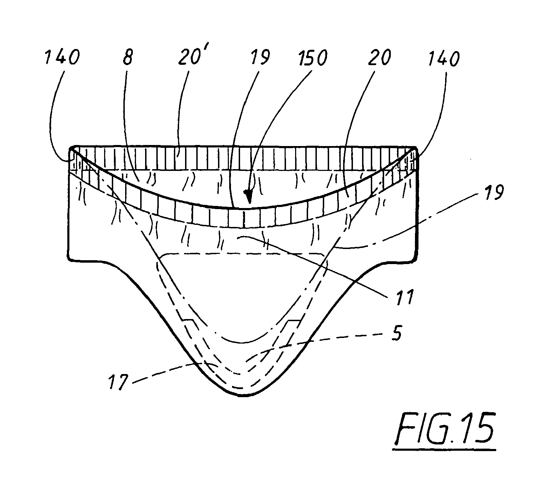

In Figure 15, an illustrative embodiment is shown which is somewhat modified in relation to the embodiment according to Figures 13 and 14. Components which are corresponded to by the same or similar parts as in Figures 13 and 14 have been provided with the same reference notations. Compared with the illustrative embodiment according to Figure 14, the pants-like product according to Figure 15 has a more conventional pants cut. The fundamental difference is the low cut of the front section 11 of the pants. This has been marked in the figure with the reference numeral 150. The low cut at the front at 150 makes it easier for the upper limit edge of the front section to be pulled down to a position below the penis of the user. In order to reduce the risk of the pants being pulled down in their entirety as the front section 11 is pulled down by the user in a visit to a urinal, the waist section 20 can at the front have higher elastic stretchability than the rear section 20' of the waist elastic.

The invention is not limited to the above-stated illustrative embodiments, but rather a number of modifications are possible within the scope of the following patent claims.

In the illustrative embodiments shown above, the absorption body is essentially triangular and tapered in the direction of the crotch section of the product. An absorption body which tapers in the direction of the crotch section is comfortable for the user, but rectangular absorption bodies which are not tapered in the said direction are also conceivable within the scope of the present invention.

## Claims

1. Absorbent product for men, comprising an absorption body (5) which tapers preferably towards one end from a front section (11) of the product towards a crotch section (12) of the product and which is enclosed in a sheath consisting of a liquid-tight layer (4) on the side of the absorption body facing away from the user during use and a liquid-permeable layer (3) on the opposite side of the absorption body (5), both of which layers extend beyond the absorption body round about this and are mutually joined there, and the absorption body (5) is arranged to extend, during product usage, from the said front section (11) of the product in the direction of the crotch section (12) and is intended to extend with its narrower end section to slightly below the penis of the user, **characterized in that** on the liquid-permeable layer (3), at the narrower end section of the absorption body, a liquid barrier (17) is applied, said liquid barrier is positioned such that during usage, it is situated below the penis of the user and is curved and convex in the direction of the crotch section to prevent urine emitted by the user from leaking from the surface of the absorption body (5) towards the crotch region of the user.

2. Absorbent product according to Claim 1, **characterized in that** the said absorbent product is constituted by an insert intended for use in combination with underpants and **in that**, to this end, the product is provided with one or more fastening members on the outer side of the liquid-tight layer (4), which fastening members are intended to hold the absorbent product in its intended place inside the pants.

3. Absorbent product according to Claim 1, **characterized in that** the product as a whole has a pants-like shape having a front section (11), a rear section (8) and a crotch section (12), the front section (11) and rear section (8) being intended to surround the waist of the user, and the front and/or rear section is provided with waist elastic, which is intended to hold an applied product in place on the user.

4. Absorbent product according to Claim 3, **characterized in that** the front section (11) has at least one elastic member (20), which, during product usage, enables the front section (11) of the product to be pulled down, counter to the action of the said elastic member (20), to a position in which the upper limit edge (19) of the front section in the middle region of the front section is situated below the penis of the user, at the same time as the upper limit edge of the front section in the two outer edge regions (140) of the front section is arranged to be held in place around the waist of the user, **in that** the absorption body (5) is configured with one or more deformation zones (130, 131), which enable those parts of the absorption body which, during product usage, are situated above and over the penis of the user to be drawn down together with the rest of the front section when the front section (11) of the product is pulled down, and **in that** the front section of the product and the absorption body (5) are arranged to be returned by the elastic member (20) to their original usage position.

5. Absorbent product according to Claim 3 or 4, **characterized in that** the said waist elastic is formed from an elastic first piece (1) which, in the extended state, is essentially rectangular and which is intended to partially surround the trunk of the user and form the rear section (8) and side sections (9. 10) of the pants-like product, **in that** a second piece (2), incorporated in the product, is configured to form the front section (11) and crotch section (12) of the pants-like product, **in that** the said second piece is elongated with two opposing end edges and two opposing longitudinal edges, **in that** the width of the second piece (2), at least at the crotch section (12), is less than the length of the first piece (1), **in that** the second piece (2) with its longitudinal direction is arranged perpendicularly to the longitudinal direction of the first piece (1) and is connected by a first end section (6) to the one longitudinal edge section (7) of the first piece and centrally on this, **in that** the one end section (13) of the first piece (1) is connected to a first side edge section (14) of the second piece, and **in that** the second end section (15) of the first piece is correspondingly connected to a second side edge section (16) of the second piece (2), and the absorption body (5) is applied, in its entirety, on the second piece.

6. Absorbent product according to any of the preceding claims, **characterized in that** the said liquid barrier (17) is arranged to follow the contour of the lower, narrower end section of the absorption body (5) and is applied in its entirety inside the said contour.

7. Absorbent product according to any of Claims 1-5, **characterized in that** the liquid barrier (17) crosses the absorption body (5) close to its narrower end.

8. Absorbent product according to Claim 7, **characterized in that** the liquid barrier (17) extends in the lateral direction beyond the absorption body (5) and in the transverse direction spans the whole of the absorbent product.

9. Absorbent product according to any of the preceding claims, **characterized in that** the liquid barrier (17), at least during product usage and at least close to the middle of the absorption body (5) in the transverse direction, has a height of at least 5 mm, preferably 10-20 mm.

10. Absorbent product according to any of the preceding claims, **characterized in that** the liquid barrier (17) is fixed in the rest of the absorbent product only along its outer edge section (170) and **in that** inner-situated sections of the liquid barrier (17) are arranged to be raised from the liquid-permeable layer (3) during product usage.

11. Absorbent product according to any of Claims 1-9, **characterized in that** the liquid barrier (17) is constituted by a roll formed from one or more band-shaped materials, which roll is bent into the desired convex shape in the direction of the narrower end of the absorption body (5).

12. Absorbent product according to any of Claims 1-10, **characterized in that** the liquid barrier (17) is constituted by a number of circumferential folds of one or more band-shaped materials, which liquid barrier, following the formation of the said folds, is elongated in the direction of the fold lines (93, 94) forming the folds, and **in that** the liquid barrier is folded or bent into the desired shape.

13. Absorbent product according to Claim 12, **characterized in that** the elongated liquid barrier (17) is folded in the middle along a transverse oblique line, such as at an angle of 45°, in relation to the longitudinal direction to form a V-shaped liquid barrier with the point of the V against the crotch section.

14. Absorbent product according to Claim 12, **characterized in that** the elongated liquid barrier (17) is folded at two places along transverse lines along an acute angle (α), viewed from the lower limit edge of the liquid barrier in the applied position, to form an essentially U-shaped liquid barrier with the base of the U against the crotch section, the said acute angle preferably being less than 45°.

15. Absorbent product according to any of Claims 11-14, **characterized in that** the band-shaped material consists of non-woven, preferably hydrophobic non-woven material, or of a laminate of non-woven material and a plastics film.

16. Absorbent product according to Claim 10, **characterized in that** the liquid barrier (17) is constituted by a single material strip, for example a hydrophobic non-woven material, which has been folded or bent into suitable shape before being applied.

17. Absorbent product according to any of Claims 12-16, **characterized in that** the liquid barrier (17), on its free longitudinal edge section, is provided with a pretensioned longitudinal elastic element, such as an elastic thread (91, 92, 100), which element is intended to hold the liquid barrier in the raised state during product usage.

18. Absorbent product according to any of Claims 12-16, **characterized in that** the liquid barrier (17) has an inherent stiffness of such magnitude that a liquid barrier applied in the folded or bent state, by virtue of its own inherent stiffness in the said bent or folded state during product usage, is held with its free longitudinal edge section in the raised state.

19. Absorbent product according to any of Claims 1-9, **characterized in that** the absorption body (5) is formed from cellulose fluff, possibly with highly absorbent material mixed in, and **in that** the liquid barrier (17) is constituted by a moulding, which is formed from air-laid fibres such as cellulose fluff, and **in that** this moulding is applied so that it follows the contour of the narrower end section of the absorption body (5) on or directly outside this.

20. Absorbent product according to any of Claims 1-9. **characterized in that** the liquid barrier (17) is constituted by a foam material which has been cast or folded into the desired shape.

## Patentansprüche

1. Absorptionsprodukt für Männer, umfassend einen Absorptionskörper (5), der sich bevorzugt in Richtung eines Endes von einem Frontabschnitt (11) aus in Richtung eines Schrittabschnitts (12) des Produkts verjüngt und der in einer Hülse eingeschlossen ist, die aus einer flüssigkeitsdichten Lage (4) an der Seite des Absorptionskörpers, die während der Benutzung vom Benutzer weg gerichtet ist, und einer flüssigkeitsdurchlässigen Lage (3) auf der gegenüberliegenden Seite des Absorptionskörpers (5) besteht, wobei sich beide Lagen über den Absorptionskörper hinaus um ihn herum erstrecken und dort miteinander verbunden sind und der Absorptionskörper (5) dazu angeordnet ist, sich während der Benutzung des Produkts von dem Frontabschnitt (11) des Produkts in die Richtung des Schrittabschnitts (12) zu erstrecken und dazu gedacht ist, sich mit seinem schmaleren Endabschnitt bis leicht unterhalb des Penis des Benutzers zu erstrecken, **dadurch gekennzeichnet, dass** an der flüssigkeitsdurchlässigen Lage (3) an dem schmaleren Endabschnitt des Absorptionskörpers eine Flüssigkeitsbarriere (17) angebracht ist, wobei die Flüssigkeitsbarriere so positioniert ist, dass sie während ihrer Benutzung unterhalb des Penis des Benutzers angeordnet ist und in Richtung des Schrittabschnitts gekrümmt und konvex ist, um Urin, das von dem Benutzer abgegeben wird, daran zu hindern, aus der Oberfläche des Absorptionskörpers (5) in Richtung des Schrittbereichs des Benutzers auszulaufen.

2. Absorptionsprodukt nach Anspruch 1, **dadurch gekennzeichnet, dass** das Absorptionsprodukt durch eine Einlage gebildet wird, die zur Verwendung in Kombination mit Unterhosen gedacht ist, und dass das Produkt an diesem Ende mit einem oder mehreren Befestigungselementen an der Außenseite der flüssigkeitsdichten Schicht (4) versehen ist, welche Befestigungselemente dazu gedacht sind, das Absorptionsprodukt an seinem vorgesehenen Ort innerhalb der Hosen zu halten.

3. Absorptionsprodukt nach Anspruch 1, **dadurch gekennzeichnet, dass** das Produkt als Ganzes eine hosenähnliche Form mit einem Frontabschnitt (11), einem Rückabschitt (8) und einem Schrittabschnitt (12) aufweist, wobei der Frontabschnitt (11) und der Rückabschnitt (8) dazu gedacht sind, die Taille des Benutzers zu umgeben und der Front- und/oder Rückabschnitt mit einer Taillenelastik versehen ist, die dazu gedacht ist, ein angebrachtes Produkt an dem Benutzer zu halten.

4. Absorptionsprodukt nach Anspruch 3, **dadurch gekennzeichnet, dass** der Frontabschnitt (11) zumindest ein elastisches Element (20) aufweist, das während der Produktbenutzung den Frontabschnitt (11) des Produkts gegen die Wirkung des elastischen Elements (20) bis zu einer Position herunterzuziehen erlaubt, in der die obere Randkante (19) des Frontabschnitts in dem mittleren Bereich des Frontabschnitts unterhalb des Penis des Benutzers angeordnet ist, wobei gleichzeitig die obere Randkante des Frontabschnitts in den zwei äußeren Kantenregionen (140) des Frontabschnitts so angeordnet ist, dass sie um die Taille des Benutzers gehalten wird, dass der Absorptionskörper (5) mit einer oder mehreren deformierbaren Zonen (130, 131) ausgestaltet ist, so dass diejenigen Teile des Absorptionskörpers, die während der Produktbenutzung oberhalb und über dem Penis des Benutzers liegen, zusammen mit dem Rest des Frontabschnitts heruntergezogen werden können, wenn der Frontabschnitt (11) des Produkts heruntergezogen wird, und dass der Frontabschnitt des Produkts und der Absorptionskörper (5) dazu angeordnet sind, durch das elastische Element (20) in ihre ursprüngliche Benutzungsposition zurückgeführt zu werden.

5. Absorptionsprodukt nach Anspruch 3 oder 4, **dadurch gekennzeichnet, dass** die Taillenelastik aus einem elastischen ersten Stück (1) gebildet ist, das im ausgedehnten Zustand im Wesentlichen rechteckig ist und das dazu gedacht ist, den Rumpf des Benutzers teilweise zu umgeben und den Rückabschnitt (8) und Seitenabschnitte (9, 10) des hosenähnlichen Produkts zu bilden, dass ein zweites Stück (2), das in das Produkt eingearbeitet ist, dazu ausgestaltet ist, den Frontabschnitt (11) und Schrittabschnitt (12) des hosenähnlichen Produkts zu bilden, dass das zweite Stück mit zwei einander gegenüberliegenden Endkanten und zwei einander gegenüberliegenden Längskanten verlängert ist, dass die Breite des zweiten Stücks (2) zumindest an dem Schrittabschnitt (12) kleiner als die Länge des ersten Stücks (1) ist, dass das zweite Stück (2) mit seiner Längsrichtung senkrecht zu der Längsrichtung des ersten Stücks (1) angeordnet ist und durch einen ersten Endabschnitt (6) mit dem einen Längskantenabschnitt (7) des ersten Stücks und zentral darauf verbunden ist, dass der eine Endabschnitt (13) des ersten Stücks (1) mit einem ersten Seitenkantenabschnitt (14) des zweiten Stücks verbunden ist und dass der zweite Endabschnitt (15) des ersten Stücks entsprechend mit einem zweiten Seitenkantenabschnitt (16) des zweiten Stücks (2) verbunden ist und der Absorptionskörper (5) vollständig an dem zweiten Stück angebracht ist.

6. Absorptionsprodukt nach einem der vorhergehenden Ansprüche, **dadurch gekennzeichnet, dass** die Flüssigkeitsbarriere (17) so angeordnet ist, dass sie dem Umriss des unteren, schmaleren Endabschnitts des Absorptionskörpers (5) folgt und vollständig innerhalb des Umrisses angebracht ist.

7. Absorptionsprodukt nach einem der Ansprüche 1 bis 5, **dadurch gekennzeichnet, dass** die Flüssigkeitsbarriere (17) den Absorptionskörper (5) nah an seinem schmaleren Ende kreuzt.

8. Absorptionsprodukt nach Anspruch 7, **dadurch gekennzeichnet, dass** sich die Flüssigkeitsbarriere (17) in der seitlichen Richtung über den Absorptionskörper (5) hinaus erstreckt und in der Querrichtung das gesamte Absorptionsprodukt überspannt.

9. Absorptionsprodukt nach einem der vorhergehenden Ansprüche, **dadurch gekennzeichnet, dass** die Flüssigkeitsbarriere (17) zumindest während der Produktbenutzung und zumindest nah an der Mitte des Absorptionskörpers (5) in der Querrichtung eine Höhe von mindestens 5 mm, bevorzugt 10-20 mm aufweist.

10. Absorptionsprodukt nach einem der vorhergehenden Ansprüche, **dadurch gekennzeichnet, dass** die Flüssigkeitsbarriere (17) im Rest des Absorptionsprodukts nur entlang ihres äußeren Kantenbereichs (170) befestigt ist und dass weiter innen angeordnete Abschnitte der Flüssigkeitsbarriere (17) dazu angeordnet sind, aus der flüssigkeitsdurchlässigen Lage (3) während der Produktbenutzung angehoben zu werden.

11. Absorptionsprodukt nach einem der Ansprüche 1 bis 9, **dadurch gekennzeichnet, dass** die Flüssigkeitsbarriere (17) durch eine Rolle gebildet ist, die aus einem oder mehreren bandförmigen Materialien geformt ist, welche Rolle in die gewünschte konvexe Form in der Richtung des schmaleren Endes des Absorptionskörpers (5) gebogen ist.

12. Absorptionsprodukt nach einem der Ansprüche 1 bis 10, **dadurch gekennzeichnet, dass** die Flüssigkeitsbarriere (17) durch mehrere umfangseitige Falten eines oder mehrerer bandförmiger Materialien gebildet ist, welche Flüssigkeitsbarriere der Formation der Falten folgend in der Richtung der Faltelinien (93, 94), die die Falten bilden, verlängert ist und dass die Flüssigkeitsbarriere in die gewünschte Form gefaltet oder gebogen ist.

13. Absorptionsprodukt nach Anspruch 12, **dadurch gekennzeichnet, dass** die verlängerte Flüssigkeitsbarriere (17) in der Mitte entlang einer schrägen Querlinie, z.B. in einem Winkel von 45°, in Bezug auf die Längsrichtung gefaltet ist, um eine V-förmige Flüssigkeitsbarriere zu bilden, wobei die Spitze des V gegen den Schrittabschnitt gerichtet ist.

14. Absorptionsprodukt nach Anspruch 12, **dadurch gekennzeichnet, dass** die verlängerte Flüssigkeitsbarriere (17) an zwei Stellen entlang von Querlinien entlang eines spitzen Winkels (α), gesehen von der unteren Randkante der Flüssigkeitsbarriere in der angebrachten Position, gefaltet ist, um eine im Wesentlichen U-förmige Flüssigkeitsbarriere zu bilden, wobei die Basis des U gegen den Schrittabschnitt gerichtet ist, wobei der spitze Winkel bevorzugt kleiner als 45° ist.

15. Absorptionsprodukt nach einem der Ansprüche 11 bis 14, **dadurch gekennzeichnet, dass** das bandförmige Material aus Vlies, bevorzugt hydrophobem Vliesmaterial, oder aus einem Laminat aus Vliesmaterial und einer Kunststofffolie besteht.

16. Absorptionsprodukt nach Anspruch 10, **dadurch gekennzeichnet, dass** die Flüssigkeitsbarriere (17) durch einen einzigen Materialstreifen, z.B. eines hydrophoben Vliesmaterials, gebildet ist, das in eine geeignete Form gefaltet oder gebogen wurde, bevor es angebracht wurde.

17. Absorptionsprodukt nach einem der Ansprüche 12 bis 16, **dadurch gekennzeichnet, dass** die Flüssigkeitsbarriere (17) an ihrem freien Längskantenabschnitt mit einem vorgespannten längselastischen Element wie einem elastischen Faden (91, 92, 100) versehen ist, welches Element dazu gedacht ist, die Flüssigkeitsbarriere während der Produktbenutzung in dem angehobenen Zustand zu halten.

18. Absorptionsprodukt nach einem der Ansprüche 12 bis 16, **dadurch gekennzeichnet, dass** die Flüssigkeitsbarriere (17) eine inhärente Steifigkeit solcher Größenordnung aufweist, dass eine Flüssigkeitsbarriere, die in dem gefalteten oder gebogenen Zustand angebracht wird, durch ihre eigene inhärente Steifigkeit in dem gebogenen oder gefalteten Zustand während der Produktbenutzung mit ihrem Längskantenabschnitt in dem angehobenen Zustand gehalten wird.

19. Absorptionsprodukt nach einem der Ansprüche 1 bis 9, **dadurch gekennzeichnet, dass** der Absorptionskörper (5) aus Fluffzellstoff, möglicherweise mit eingemischtem hochabsorbierendem Material, gebildet ist und dass die Flüssigkeitsbarriere (18) durch eine Form gebildet ist, die aus luftgelegenen Fasern, z.B. Fluffzellstoff, gebildet ist, und dass diese Form so angebracht ist, dass sie dem Umriss des schmaleren Endabschnitts des Absorptionskörpers (5) an oder direkt außerhalb von ihm folgt.

20. Absorptionsprodukt nach einem der Ansprüche 1 bis 9, **dadurch gekennzeichnet, dass** die Flüssigkeitsbarriere (17) durch ein Schaummaterial gebildet ist, das in die gewünschte Form gegossen oder gefaltet ist.

## Revendications

1. Article absorbant destiné aux hommes, comprenant un corps d'absorption (5) qui se rétrécit de préférence en direction d'une des extrémités à partir d'une zone avant (11) de l'article, en direction d'une zone d'entrejambe (12) de l'article et qui est enfermé dans une gaine constituée d'une couche (4) étanche aux liquides située sur le côté du corps d'absorption orienté à l'écart de l'utilisateur lors de l'utilisation et d'une couche (3) perméable aux liquides située sur le côté opposé du corps d'absorption (5), les deux couches s'étendant au-delà du corps d'absorption de manière arrondie autour de celui-ci et y étant mutuellement assemblées, et le corps d'absorption (5) est agencé de manière à s'étendre, lors de l'utilisation du produit, à partir de ladite zone avant (11) de l'article en direction de la zone d'entrejambe (12) et est destiné à s'étendre par sa partie d'extrémité plus étroite jusque légèrement sous le pénis de l'utilisateur, **caractérisé en ce que** sur la couche (3) perméable aux liquides au niveau de la partie d'extrémité plus étroite du corps d'absorption, est appliquée une barrière (17) pour les liquides, ladite barrière pour les liquides étant positionnée de telle manière que, lors de l'utilisation, elle se situe sous le pénis de l'utilisateur et présente une courbure convexe en direction de la zone d'entrejambe afin d'empêcher que de l'urine émise par l'utilisateur fuie depuis la surface du corps d'absorption (5) en direction de la zone d'entrejambe de l'utilisateur.

2. Article absorbant selon la revendication 1, **caractérisé en ce que** ledit article absorbant est constitué d'un insert destiné à être utilisé en combinaison avec une culotte et **en ce que** l'article est, dans ce but, muni d'un ou de plusieurs éléments d'attache sur le côté extérieur de la couche imperméable aux liquides (4), lesquels éléments d'attache sont destinés à maintenir l'article absorbant à l'emplacement souhaité à l'intérieur de la culotte.

3. Article absorbant selon la revendication 1, **caractérisé en ce que** l'article dans son ensemble a une forme analogue à celle d'une culotte, comportant une zone avant (11), une zone arrière (8) et une zone d'entrejambe (12), la zone avant (11) et la zone arrière (8) étant destinées à entourer la taille de l'utilisateur, et la zone avant et/ou la zone arrière étant munie d'un élastique pour la taille qui est destiné à maintenir en place un article qui est appliqué sur l'utilisateur.

4. Article absorbant selon la revendication 3, **caractérisé en ce que** la zone avant (11) comporte au moins un élément élastique (20) qui, lors de l'utilisation de l'article, permet que la zone avant (11) de l'article soit tirée vers le bas à l'encontre de l'action dudit élément élastique (20) jusqu'à un emplacement dans lequel le bord (19) formant la limite supérieure de la zone avant dans la région médiane de la zone avant est situé sous le pénis de l'utilisateur, en même temps que le bord formant la limite supérieure de la zone avant dans les deux régions de bord extérieur (14) de la zone avant est agencée de manière à être maintenue en place autour de la taille de l'utilisateur, **en ce que** le corps d'absorption (5) a une configuration comportant une ou plusieurs zones de déformation (130, 131) qui permettent que les parties du corps d'absorption qui, lors de l'utilisation de l'article, sont situées au-dessus du et sur le pénis de l'utilisateur soient tirées vers le bas, en même temps que le reste de la zone avant lorsque la zone avant (11) de l'article est tirée vers le bas et **en ce que** la zone avant de l'article et le corps d'absorption (5) sont agencés de manière à être ramenés par l'élément élastique (20) à leur position d'utilisation d'origine.

5. Article absorbant selon la revendication 3 ou 4, **caractérisé en ce que** ledit élastique pour la taille est constitué d'une première partie élastique (1) qui à l'état étendu est essentiellement rectangulaire et qui est destinée à entourer partiellement le tronc de l'utilisateur et forme la zone arrière (8) et les zones latérales (9, 10) de l'article du genre culotte, **en ce qu'**une deuxième partie (2) incorporée dans l'article, a une configuration destinée à réaliser la zone avant (11) et la zone d'entrejambe (12) de l'article du genre culotte, **en ce que** ladite deuxième partie a une forme oblongue comportant deux bords d'extrémité opposés et deux bords longitudinaux opposés, **en ce que** la largeur de la deuxième partie (2), au moins au niveau de la zone d'entrejambe (12) est inférieure à la longueur de la première partie (1), **en ce que** la direction longitudinale de la deuxième partie (2) est agencée perpendiculairement à la direction longitudinale de la première partie (1) et est reliée par une première zone d'extrémité (6) à la zone de bord longitudinal (7) de la première partie (1) et centralement à celle-ci, **en ce qu'**une zone d'extrémité (13) de la première partie (1) est connectée à une première zone de bord latéral (14) de la deuxième partie, et **en ce que** la deuxième zone d'extrémité (15) de la première partie est reliée de manière correspondante à une deuxième zone de bord latéral (16) de la deuxième partie (2) et **en ce que** le corps d'absorption (5) est appliqué en totalité sur la deuxième partie.

6. Article absorbant selon l'une quelconque des revendications qui précèdent, **caractérisé en ce que** ladite barrière (17) pour les liquides est agencée de manière à suivre le contour de la zone d'extrémité inférieure plus étroite du corps d'absorption (5) et est appliquée en totalité à l'intérieur dudit contour.

7. Article absorbant selon l'une quelconque des revendications 1 à 5, **caractérisé en ce que** la barrière (17) pour les liquides est en travers du corps d'absorption (5) tout près de son extrémité plus étroite.

8. Article absorbant selon la revendication 7, **caractérisé en ce que** la barrière (17) pour les liquides s'étend dans la direction latérale au-delà du corps d'absorption (5) et s'étend sur la totalité de l'article absorbant dans la direction transversale.

9. Article absorbant selon l'une quelconque des revendications qui précèdent, **caractérisé en ce que** la barrière (17) pour les liquides, au moins pendant l'utilisation de l'article et au moins tout près du milieu du corps d'absorption (5) a, dans la direction transversale, une hauteur d'au moins 5 mm, de préférence de 10 à 20 mm.

10. Article absorbant selon l'une quelconque des revendications qui précèdent, **caractérisé en ce que** la barrière (17) pour les liquides est, dans le reste du corps d'absorption, fixée uniquement le long de sa zone de bord extérieur (170) et **en ce que** les parties de la barrière (17) pour liquides situées à l'intérieur sont agencées de manière à être dressées à partir de la couche (3) perméable aux liquides lors de l'utilisation de l'article.

11. Article absorbant selon l'une quelconque des revendications 1 à 9, **caractérisé en ce que** la barrière (17) pour les liquides est constituée d'un rouleau réalisé à partir d'un ou de plusieurs matériaux en forme de bande, lequel rouleau est courbé de manière à prendre la forme convexe désirée en direction de l'extrémité plus étroite du corps d'absorption (5).

12. Article absorbant selon l'une quelconque des revendications 1 à 10, **caractérisé en ce que** la barrière (17) pour les liquides est constituée d'un certain nombre de plis circonférentiels d'un ou de plusieurs matériaux en forme de bande, laquelle barrière pour les liquides, à la suite de la réalisation desdits plis est étendue dans la direction des lignes de pliage (93, 94) qui forment les plis et **en ce que** la barrière pour les liquides est pliée ou courbée de manière à prendre la forme désirée.

13. Article absorbant selon la revendication 12, **caractérisé en ce que** la barrière (17) pour les liquides à l'état étendu est pliée en son milieu le long d'une ligne oblique transversale, par exemple selon un angle de 45°, par rapport à la direction longitudinale afin de réaliser une barrière pour les liquides en forme de V, la pointe du V étant située contre la zone d'entrejambe.

14. Article absorbant selon la revendication 12, **caractérisé en ce que** la barrière (17) pour les liquides à l'état étendu est pliée à deux endroits le long de lignes transversales selon un angle aigu (α), lorsque l'on observe depuis le bord constituant la limite inférieure de la barrière pour liquides en position d'application, afin de réaliser une barrière pour liquides sensiblement en forme de U, la base de l'U étant située contre la zone d'entrejambe, ledit angle aigu étant de préférence inférieur à 45°.

15. Article absorbant selon l'une quelconque des revendications 11 à 14, **caractérisé en ce que** le matériau en forme de bande consiste en un matériau non tissé, de préférence hydrophobe, ou en un lamellé de matériau non tissé et d'un film de matière plastique.

16. Article absorbant selon la revendication 10, **caractérisé en ce que** la barrière (17) pour les liquides est constituée d'une unique petite bande de matériau, par exemple d'un matériau non tissé hydrophobe, qui a été plié ou courbé de manière à prendre une forme convenable avant d'être appliquée.

17. Article absorbant selon l'une quelconque des revendications 12 à 16, **caractérisé en ce que** la barrière (17) pour les liquides est munie, sur sa partie de bord longitudinal libre, d'un élément élastique longitudinal sous pré-tension, tel qu'un fil élastique (91, 92, 100), lequel élément est destiné à maintenir la barrière pour les liquides à l'état dressé pendant l'utilisation de l'article.

18. Article absorbant selon l'une quelconque des revendications 12 à 16, **caractérisé en ce que** la barrière (17) pour les liquides a une rigidité inhérente d'une telle ampleur qu'une barrière pour les liquides appliquée à l'état plié ou courbé est maintenue, lors de l'utilisation de l'article en raison de sa propre rigidité inhérente, à l'état plié ou courbé, de façon telle que sa partie de bord longitudinal libre est à l'état dressé.

19. Article absorbant selon l'une quelconque des revendications 1 à 9, **caractérisé en ce que** le corps d'absorption (5) est constitué de pâte de cellulose, comportant éventuellement en mélange un matériau fortement absorbant, et **en ce que** la barrière (17) pour les liquides est réalisée sous forme d'un moulage qui est formé à partir de fibres déposées au moyen d'air telles que des fibres de pâte de cellulose et **en ce que** le moulage est appliqué de manière à suivre le contour de la zone d'extrémité plus étroite du corps d'absorption (5) sur, ou directement à l'extérieur de celle-ci.

20. Article absorbant selon l'une quelconque des revendication 1 à 9, **caractérisé en ce que** la barrière (17) pour les liquides est constituée d'un matériau du type mousse qui a été coulé ou moulé pour prendre la forme désirée.
